# EUROPEAN PATENT SPECIFICATION

(11) **EP 3 352 897 B1**
(45) Date of publication and mention of the grant of the patent: **19.08.2020**
(21) Application number: 16738767.9
(22) Date of filing: 13.07.2016
(51) Int. Cl.: B01J 20/34, C07C 7/00, C07C 7/04, C10G 25/03, C10G 25/12, C10G 57/02, C07C 2/12, C07C 7/13, C07C 7/163

(54) **A PROCESS FOR REGENERATING AN ADSORBENT FOR NITROGEN-CONTAINING COMPOUNDS PRESENT IN A HYDROCARBON FEED**
VERFAHREN ZUR REGENERATION EINES ADSORPTIONSMITTELS FÜR STICKSTOFFHALTIGE VERBINDUNGEN IN EINEM KOHLENWASSERSTOFFSTROM
PROCÉDÉ DE RÉGÉNÉRATION D'UN ADSORBANT DE COMPOSÉS CONTENANT DE L'AZOTE PRÉSENTS DANS UNE CHARGE D'HYDROCARBURE

(30) Priority: 24.09.2015 EP 15186623
(43) Date of publication of application: 01.08.2018
(73) Proprietor: ExxonMobil Chemical Patents Inc., Baytown, TX 77520-2101 (US)
(72) Inventor: CARRETTIN, Silvio, 1950 Kraainem (BE); MARTENS, Luc, 1860 Meise (BE); HAMILTON, Paul, Spring, TX 77389 (GB); TAYLOR, Christopher, Southampton Hampshire SO15 2RZ (GB); JANSSEN, Marcel, 3010 Kessel-Lo (BE); DE SMIT, Emiel, 1200 Sint-Lambrechts-Woluwe (BE); DENAYER, Joeri, 1050 Brussels (BE); LIEKENS, Anuschka, 1050 Brussels (BE); SMITS, Marianne, 2640 Mortsel (BE); WELFORD, Mark, Southampton Hampshire SO15 7NS (GB)
(74) Representative: ExxonMobil Chemical Europe Inc.
(86) International application number: PCT/EP2016/066645
(87) International publication number: WO 2017/050454

(56) References cited:
- US-A- 5 271 835
- US-A- 6 019 887
- US-A1- 2005 137 442
- US-A1- 2010 268 008

## Description

The present invention relates to a process for regenerating an adsorbent for nitrogen-containing compounds present in a hydrocarbon feed, such as a feed containing light olefins, *i.e.,* typically C₃ to C₆ olefins, optionally containing at least one of diolefins and cyclic olefins, wherein the adsorbent used to remove the nitrogen-containing compound(s) from the feed is regenerated in a multiple-step procedure such as a three-step procedure comprising contacting the adsorbent with a stream comprising an inert gas such as nitrogen or a saturated hydrocarbon combined with water, at different temperatures. The present invention also relates to a process for converting a hydrocarbon feed contaminated with at least one nitrogen containing compound into a hydrocarbon product, said process comprising the use and regeneration of an adsorbent as defined herein.

### Background

Hydrocarbon feeds containing light olefins, *i.e.,* typically C₃ to C₆ olefins are used in catalytic oligomerization processes to obtain oligomers and/or polymers, typically octenes, nonenes and dodecenes. These products may be converted to further products such as alcohols, plasticizers, adipates, mercaptans and solvents.

The hydrocarbon feed streams are derived from various sources including refinery operations such as catalytic crackers and steam crackers and are known to contain certain amounts of impurities including but not limited to nitrogen containing compounds such as nitriles. These impurities may have an adverse effect on the catalysts used in the oligomerization process such as phosphoric acid-based catalysts, zeolite-based catalysts and supported metal catalysts, *i.e.,* they may act as catalyst poisons (contaminants) reducing the activity and service life of the catalysts.

Prior art approaches to remove nitrogen-containing contaminants include removal via liquid-liquid extraction techniques or via adsorption techniques using a so-called guard bed.

With regard to the use of guard beds, a number of approaches have been developed in the prior art. For example, WO-A-2013/013885 describes a continuous process for converting a hydrocarbon feed, such as a feed containing light olefins, contaminated with nitrile compounds, into a hydrocarbon product, the process comprising the steps of, in a first adsorber, contacting a hydrocarbon feed comprising nitriles with at least one adsorbent in order to remove nitriles from the feed; converting the feed with a reduced level of nitriles into a hydrocarbon product; switching the flow of hydrocarbon feed comprising nitriles from the first adsorber to a second adsorber, and contacting the hydrocarbon feed comprising nitriles with at least one adsorbent in said second adsorber in order to remove nitriles from the feed; and, desorbing the nitriles adsorbed on the at least one adsorbent of the first adsorber with a portion of the hydrocarbon products. Thus, the process of WO-A-2013/013885 uses a specific adsorber arrangement and associated handling of feed and process streams to achieve a reduced level of nitriles in the feed stream for a conversion reaction such as olefin oligomerization. In the background of the invention section of WO-A-2013/013885, it is mentioned that a spent guard bed may be regenerated by heating under a flow of nitrogen or under a flow of a hydrocarbon which is free of nitrile contaminants.

US-A-2008/0194902 relates to the purification of diene-contaminated liquid and gas streams by treating these streams with adsorbents comprising single or multiple transition metal polycation-exchanged faujasites having a certain silicon to aluminum ratio, wherein the transition metal polycations have the general formula [Tr_{α}O_{β}]ⁿ⁺, wherein α varies from 2 to 8, β varies from 0 to 4, and n varies from 1 to 3, and wherein said transition metals is preferably selected from the group consisting of copper, cadmium, zinc, manganese, nickel and iron. In this context, a regeneration procedure for the adsorbent is described in which the adsorbent is first rinsed with hexene-1 at ambient temperature and at the same flow rate as during adsorption followed by subjecting the adsorbent bed to a hydrogen flow at a rate of 100 ml/min and at a gradually raised temperature from ambient up to 180°C.

US -A-2010/0268008 relates to regeneration of adsorbents employed to treat hydrocarbon feeds by subjecting the adsorbent at elevated temperatures to a flow of substantially inert gas(i.e., inert relative to the adsorbent) such as nitrogen, air, natural gas, liquefied petroleum gas, methane, ethane, propane, butanes, pentanes, or steam, or to a flow of a substantially inert liquid such as liquefied petroleum gas, ethane, propane, butanes, pentanes, hexanes, benzene, toluene, or xylenes.

Also, when using a guard bed to remove nitrogen-based impurities such as nitriles, other impurities present in the feed may detrimentally affect the efficient removal of the nitrogen-based impurities. For example, diolefins and/or cyclic olefins may have an affinity to the material used as the adsorbent in a guard bed intended to remove nitrogen-based impurities. This will result in competitive adsorption thereby reducing the adsorption capacity and run time of a given adsorbent used in the guard bed. Furthermore, coke formation due to the reaction of diolefins (linear and cyclic) present in a stream during adsorption and desorption may be a problem.

Thus, there remains a need for further processes which allow for an efficient removal of nitrogen-based impurities while allowing at the same time for increased run time of the process and process materials such as the adsorbents for the nitrogen contaminants.

### Summary of the Invention

According to a first aspect, the present invention solves the above problem(s) by a process for regenerating an adsorbent for nitrogen-containing compounds present in a hydrocarbon feed, said process comprising the steps of
a) contacting the adsorbent with a stream comprising an inert gas and water at a temperature in the range of from 10 to 60°C,
b) followed by contacting the adsorbent with a stream comprising an inert gas and water at an elevated temperature in the range of from 200 to 260°C;
c) followed by cooling the adsorbent in an inert gas, wherein the inert gas is selected from the group consisting of nitrogen, saturated hydrocarbons and mixtures thereof and wherein the streams used in steps (a) and (b) are water saturated inert gas.

According to one embodiment the stream used in steps (a), and (b) comprises at least 96 wt %, preferably at least 98 wt%, more preferably at least 99 wt% of at least one inert gas.

According to another embodiment, the stream used in steps (a) and (b) comprises at least one inert gas, typically nitrogen, and water and is a water-saturated inert gas. Such process may further comprises contacting the adsorbent from step (b) in a dry inert gas, preferably dry nitrogen, at a temperature in the range of from 200 to 250°C in order to remove water from the adsorbent before cooling according to step (c). The water is usually present in the stream at a partial pressure of from 0.1 to 20 kPa, preferably from 1 to 20 kPa. Water partial pressure less than 1.5 kPa, preferably between 0.7 and 1.2 kPa provides good results.

According to another embodiment, the stream used in steps (a) and (b) comprises at least 99 wt%, preferably at least 99.5 wt% nitrogen gas and the nitrogen gas resulting from the regeneration may be washed with a hydrocarbon having a low vapor pressure, in particular with a hydrocarbon having 8 to 13 carbon atoms.

According to another embodiment, the stream comprises at least 98, preferably at least 99, and more preferably at least 99.5 wt% of one or more saturated hydrocarbons having from 1 to 16 carbon atoms. Mixtures of saturated hydrocarbon comprising from 7 to 12 carbon atoms as well as mixtures of saturated hydrocarbons comprising from 5 to 6 carbon atoms provide good results. Saturated hydrocarbon mixtures having a boiling range within the temperature range of 120 to 220°C, or 50 to 80°C may also be preferred.

It goes without saying that, depending on the process conditions used in said steps (a) and (b), the stream contains liquid in equilibrium with its gas phase.

According to a second aspect, the present invention relates to process for converting a hydrocarbon feed contaminated with at least one nitrogen-containing compound into a hydrocarbon product, said process comprising the steps of:
i) providing a hydrocarbon feed stream contaminated with at least one nitrogen-containing compound;
ii) contacting the feed stream with an adsorbent to remove the nitrogen-containing compound(s) from the feed;
iii) contacting the feed stream having a reduced level of nitrogen-containing compounds with a catalyst in order to convert the feed stream into a hydrocarbon product;
iv) regenerating the adsorbent; and,
v) optionally, repeating steps (i) to (iii) or steps (i) to (iv),
wherein the regeneration step (iv) is as defined in accordance with the first aspect of the present invention.

The hydrocarbon feed stream used in this invention may be contaminated with other compounds having an affinity to adsorbents such as oxygenates, sulphur-containing compounds, water, diolefins and mixtures thereof. Such hydrocarbon feed may be subjected to further treatment(s) such as distillation and/or reduction before contacting with the adsorbent to reduce the content of said other compounds.

According to other embodiments, the hydrocarbon feed stream contaminated with at least one nitrogen containing compound is an olefins containing feed that optionally contains at least one of diolefins. Such hydrocarbon feed stream is preferably further subjected to selective hydrogenation to reduce the content of diolefins before contact with the adsorbent.

In a preferred embodiment, at least one nitrogen containing compound is a nitrile, in particular propionitrile (PCN) or acetonitrile (ACN).

Further and preferred embodiments are disclosed in the dependent claims and in the following description including the examples and figures illustrating the present invention.

### Brief Description of the Figures

**Figure 1** is a graphic representation of adsorbent service life in terms of the level of nitrogen-containing contaminants present in the feed passing over the adsorbent before and after regeneration cycles using decene. (not according to the invention).
**Figure 2** is another graphic representation of adsorbent service life in terms of the level of nitrogcn-containing contaminants present in the feed passing over the adsorbent before and after regeneration cycles using decene (not according to the invention).
**Figure 3** is another graphic representation of adsorbent service life in terms of the level of nitrogen-containing contaminants present in the feed passing over the adsorbent before and after regeneration with decene and a water-saturated nitrogen stream.
**Figure 4** is another graphic representation of adsorbent service life in terms of the level of nitrogen-containing contaminants present in the feed after passing over the adsorbent before and after regeneration with a water-saturated nitrogen stream.
**Figure 5** is another graphic representation of adsorbent service life in terms of the level of nitrogen-containing contaminants present in the feed passing over the adsorbent before and after regeneration with dry nitrogen (not according to the invention).
**Figure 6** is another graphic representation of adsorbent service life in terms of the level of nitrogen-containing contaminants present in the feed passing over the adsorbent before and after regeneration with water-saturated nitrogen and using a feed subjected to hydrogenation prior to passing over the adsorbent.
**Figure 7** is another graphic representation of adsorbent service life in terms of the level of nitrogen-containing contaminants present in the feed after passing over the adsorbent before and after regeneration with water-saturated nitrogen and using a feed subjected to distillation prior to passing over the adsorbent.
**Figure 8** is a process scheme showing the disposition of nitrogen-rich regeneration gas by contacting the gas with hydrocarbons having a low vapor pressure.
**Figure 9** is a graphic representation of the adsorbent capacity after multiple regeneration runs using different regeneration schemes (not according to the invention).

### Detailed Description of the Invention

Before the materials, compounds, components, compositions and/or processes of the present invention are disclosed in more detail, it is noted that the singular forms "a", "an" and "the" include plural referents unless otherwise specified.

Furthermore, the words "comprising" (and any form of comprising such as "comprise" and "comprises"), "having" (and any form of having, such as "have" and "has"), "including" (and any form of including, such as "includes" and ("include") or "containing" (and any form of containing, such as "contains" and "contain") are inclusive or open-ended and do not exclude additional, unrecited elements such as materials, compounds or compositions or additional process steps.

The terms "feed" and "feedstock" are used herein interchangeably to refer to the hydrocarbon feed used in this invention. Typically the hydrocarbon feed contains at least one light olefins such as a C₃ to C₆ olefin. When applicable, said feed may also be referred to as "olefin feed".

The hydrocarbon feed is characterized by comprising a certain level of nitrogen containing compounds and, optionally, certain levels of other compounds such as oxygenates sulphur-containing compounds, water, diolefins, cyclic olefins and mixtures thereof, preferably diolefins and/or cyclic olefins having an affinity to adsorbents for nitrogen compounds, said adsorbents being disclosed in detail in the following description and appended claims. The levels (concentrations) of both the nitrogen and said other compounds are usually in a range referred to as impurities or at least as minor components of the stream. In particular, with regard to nitrogen and other heteroatom containing compounds, the concentrations of this type of compounds will usually be in the range of from 0.1 to several hundred weight ppm (wt ppm), typically in a range of from 10 to 500 wt ppm, relative to the total weight of the stream. With regard to non-heteroatom containing compounds, such as dienes, the concentrations of this type of compounds will typically be in the range of from 0.01 to 5 or 10 weight % (wt%) relative to the total weight of the stream. In this context, the phrase "other compounds having an affinity to adsorbents for nitrogen containing compounds" means that the respective compound interacts with an adsorbent suitable to adsorb nitrogen containing compounds such as nitriles in a manner that reduces the adsorbent's capacity for adsorbing said nitrogen containing compounds, e.g., due to competitive adsorption. The adsorption step used in this invention is capable of reducing the content of nitrogen containing compounds in the hydrocarbon feed from an initial range of from 10 to 500 wt ppm to the range of from 20 to 500 wt ppb relative to the weight of the feed.

According to one embodiment, the hydrocarbon feed containing other compounds having an affinity to the adsorbent is subjected to at least one distillation step prior to contacting with the adsorbent to provide one or more fractions having a reduced level of said other compounds and said fraction(s), optionally combined together, are contacted with the adsorbent to remove the nitrogen containing compounds.

In another embodiment of the present invention, the hydrocarbon feed contaminated with at least one nitrogen containing compound contains diolefins and/or cyclic olefins and is preferably subjected to selective hydrogenation to reduce the content of diolefins and optionally cyclic olefins before contact with the adsorbent. This embodiment is particularly used if the levels of dienes in the feed are within the range of 0.01 to 10 weight % (wt%) relative to the total weight of the stream mentioned above.

In further embodiments of the present invention, compounds, components, compositions and processes as disclosed herein can consist of the features disclosed in these respects.

### Regeneration

As stated above, according to a first aspect, the present invention solves the above problem(s) by a process for regenerating an adsorbent for nitrogen-containing compounds present in a hydrocarbon feed, said feed preferably being a olefins containing feed that optionally also contains at least one of diolefins and cyclic olefins, said process comprising the steps of
a) contacting the adsorbent with a stream comprising an inert gas and water at a temperature in the range of from 10 to 60°C, preferably 20 to 50°C, such as 40°C;
b) followed by contacting the adsorbent with a stream comprising an inert gas and water at an elevated temperature in the range of from 200 to 260°C; preferably 220 to 240°C, such as 230°C;
c) followed by cooling the adsorbent in an inert gas, preferably to a temperature at or above ambient temperature such as 40°C.

The inert gas is selected from the group consisting of nitrogen, saturated hydrocarbons and mixtures thereof. The inert gases used in each of steps (a) to (c) may be the same or may be different. Preferably the same inert gas is used in each of these steps.

According to one embodiment the stream used in step (a), and (b) consists essentially in an inert gas comprises at least 99 wt %, preferably at least 99.5 wt%, typically 99.9 wt% of at least one inert gas, typically nitrogen. The impurities in such inert gas usually, include oxygen, hydrogen, carbon monoxide and carbon dioxide. Such impurities are usually present in an amount less than 1000 ppm by weight, preferably less than 100 and more preferably less than 10 ppm by weight.

Additionally, when the gas steam used in steps (a) and (b) comprises at least 99 wt% nitrogen gas, the nitrogen gas resulting from the regeneration may be washed with a hydrocarbon having a low vapor pressure, in particular with a hydrocarbon having from 5 to 13 carbon atoms or mixtures such hydrocarbons. Octene, isooctane, octane or dodecane or mixtures thereof are advantageously used. The preferred nitrogen to hydrocarbon ratio (wt/wt) is between about 0.5 and 2.

According to the invention the stream used in steps (a) and (b) comprises at least one inert gas and water and is a water-saturated nitrogen. Such process may further comprises contacting the adsorbent from step (b) in a dry inert gas, preferably dry nitrogen, at a temperature in the range of from 200 to 250°C in order to remove water from the adsorbent before cooling according to step (c). The duration of this step is usually in the range of from 1 to 36 hours. Such a process while providing satisfactory regeneration performances may be disregarded for economic reasons.

According to another embodiment, the stream comprises at least 98, preferably at least 99, and more preferably at least 99.5 wt% of one or more saturated hydrocarbons having from 1 to 15 carbon atoms. Mixtures of saturated hydrocarbon comprising from 7 to 12 carbon atoms as well as mixture of saturated hydrocarbons comprising from 5 to 6 carbon atoms provide good results. Saturated hydrocarbon mixtures having a boiling range within the temperature range of 120 to 220°C, or 50 to 80°C are also preferred.

The elevated temperature used in step (b) is preferably in the range of from 220 to 240°C.

The flow of the stream in steps (a) and (b) is usually in the Weight Hourly Space Velocity (WHSV) range of from 0.1 to 60 hrs⁻¹, preferably from 0.5 to 40 hrs⁻¹ most preferably from 1 to 30 hrs⁻¹.

In order to obtain a stream of inert gas comprising water, preferably a water-saturated stream of inert gas, the regeneration stream can be hydrated using a water saturator. The temperature of the water saturator will determine the water partial pressure in the regeneration stream. A preferred saturator temperature is between 25 and 90°C.

Alternatively, water can be co-fed with the inert gas regeneration stream in the right proportion to saturate the regeneration stream with water in a suitable temperature range, preferably in the range of from 25 to 90°C.

The above approaches are known to a person skilled in the art. For example, with regard to water partial pressure/water solubility as a function of temperature for hydrocarbons, see, e.g., P. Schatzberg, Solubilities of Water in Several Normal Alkanes from C7 to C16, Journal of Physical Chemistry, Vol. 67, 776-770 (1963) or A. Bahadori et al., Predicting Water-Hydrocarbon Systems Mutual Solubility, Chem. Eng. Technol. 2008, 31, No. 12, 1743-1747. For inert gases such as nitrogen see, e.g., the Handbook of Chemistry and Physics, 58th Edition, pp.D-180-D-181.

Water-containing nitrogen gas is preferably provided in the form of a stream characterized by an inert gas flow of from 1 to 25 h⁻¹ WHSV.

The regeneration steps (a) to (c) of the process according to the first aspect may be conducted for a period sufficient to restore at least 75 % of the adsorbent's original adsorption capacity, as determined by the amount of contaminated feed passed over the adsorbent before the adsorbent becomes ineffective, as indicated by an increase (breakthrough) in the level of nitrogen-containing compounds in the feed after contact with the adsorbent.

For example, the regeneration steps are conducted for a total period of from 12 to 144 hours. Each of steps (a) to (c) may be conducted for a period of from 3 to 48 h, typically of from 3 to 15 hours. Periods of from 12 to 48 hours are also suitable.

The increase of temperature between step (a) and step (b) can be done continuously or stepwise.

For example, and more particularly when the stream used in steps (a) and (b) consists essentially in a saturated hydrocarbon stream, the increase of temperature preferably comprises an additional step (b') between steps (a) and (b) at a temperature in the range of 80 to 110°C. In such a case regeneration step (a) may be conducted for a period of 3 to 7 hours, and steps (b') and (b) for a period of from 4 to 8 hours.

The pressure employed during regeneration may be in the range of from about 14.5 psia to about 290 psia (100kPa to 2000 kPa), and preferably from about 14.5psia to about 145 psia (100 kPa to 1000 kPa).

According to the present invention, no further process steps are preferably conducted that contribute to regeneration of the adsorbent other than those described in the context of the first aspect of the present invention.

### Adsorbents

Examples of suitable adsorbents include alumina (aluminium oxide), preferably gamma alumina or eta alumina, molecular sieves, zeolites (acidic or cation-exchanged), activated carbons, clays, optionally impregnated with metals, metal oxides and mixed metal oxides, silica gels, resins (e.g., acid resins) and combinations thereof.

Examples of metal oxides other than alumina include tin oxide, zirconium oxide, titanium oxide, iron oxide, magnesium and tungsten oxide, silicon oxide, copper oxide, nickel oxide, zinc oxide, and mixtures thereof. The adsorbent can comprise two or more of the metal oxides listed above and in any combination.

Exemplary materials and methods for making and using adsorbers are described, for example, in EP-A-1 002 852 and US-A-2005/0137442. Furthermore, processes and adsorbers as disclosed in WO 2013/013884; WO 2013/013885; WO 2013/013886; WO 2013/013887; and WO 2013/013888 may be used. In this context, it is noted that the term "adsorber" is used to refer to a unit containing an adsorbent.

Other specific examples include without limitation molecular sieve 3A (activated 16 h @ 200oC & vacuum), molecular sieve 13X (activated 1 h @ 200oC & vacuum), ZEOLYST™ CBV 100 CY (Zeolite Na-Y, white rods (80/20 FAU/Binder)), Cameron SG6 carbon (12^{∗}40 mesh, coconut shell based BCT 4443), Cameron SG6 carbon (8^{∗}30 mesh, coconut shell based, BCT 4444), Norit GAC 830W 640316, BCT 4475 active coal, Kieselgel Fein silicagel (MN), Amberlyst 15 A, silica bound ex fr F103runE60, and Alcoa Selexsorb™.

Preferably the adsorbent comprises a zeolite with faujasite (FAU) structure optionally formulated together with a suitable binder material. The weight ratio of zeolite to binder may be in the range of from 50:50 to 90:10, such as 70:30 to 80:20. The formulated zeolite/binder adsorbent may be provided in the form of particles of desired shape, such as rod-like or sphere-like particles.

Examples of binder materials that may be employed with the molecular sieves or zeolites suitable for use in the process of the invention include active and inactive materials and synthetic or naturally occurring zeolites as well as inorganic materials such as clays, silica and/or metal oxides such as alumina. Examples of other materials include porous matrix materials such as silica-alumina, silica-magnesia, silica-zirconia, silica-thoria, silica-beryllia, silica-titania as well as ternary compositions such as silica-alumina-thoria, silica-alumina-zirconia, silica-alumina-magnesia and silica-magnesia-zirconia.

The preferred zeolites with faujasite structure comprise Zeolite X or Zeolite Y. The most preferred adsorbents include sodium Zeolite Y (NaY) and sodium Zeolite X (NaX).

The preferred zeolite adsorbents of the present invention are characterized by the fact that at least 95% of the available cation sites are occupied by sodium cations. More preferably, 99% of the available cation sites are occupied by sodium cations and even more preferably 99.9 % of the available cation sites are occupied by sodium cations. Such zeolite structure is considered by the person of ordinary skill in the art as non acidic zeolite structure.

The adsorption may be carried out at lower temperatures, such as down to -5°C, at room temperature (*i.e.,* at about 15 to 20 or 25 °C) or at elevated temperatures, and thus conveniently takes place in the range of -5 to 250°C. Preferably, adsorption is carried out in the temperature range of 20 to 80°C, such as 40°C to 60°C.

The pressure employed during adsorption may be in the range of from about 400 psig to about 4000 psig (2860 kPa to 27688 kPa), and preferably from about 500 psig to about 1500 psig (3550 kPa to 10446 kPa). The hydrocarbon feed weight hourly space velocity may be in the range of from about 0.1 hr⁻¹ to about 20 hr⁻¹, and preferably from about 0.5 hr⁻¹ to about 5 hr⁻¹.

### Hydrocarbon feed

The hydrocarbon feed provided in the processes according to the aspects of the present invention can be any hydrocarbon feed contaminated with nitrogen containing compounds, including aromatic or aliphatic hydrocarbons or a combination thereof. Whilst the process of the present invention is not limited by hydrocarbon feed or the type of process for which the hydrocarbon feed is used, preferably the process of the present invention is part of an olefin oligomerization process and the hydrocarbon feed is an olefin containing feed. In particular, the feed is a C₃, C₄ and/or C₅ olefin containing stream, more preferably a C₅ olefin containing stream.

As used herein, "olefins" refers to any unsaturated hydrocarbons having the formula CₙH₂ₙ, wherein C is a carbon atom, H is a hydrogen atom, and n is the number of carbon atoms in the olefin. According to this invention, the olefins in the feed typically have from 2 to 15 carbon atoms, such as at least 3 and no more than 8 carbon atoms, and typically at least 3 and no more than 6 carbon atoms. They are also referred to as lower or light olefins. According to a preferred embodiment, the feed is a C₃, C₄ and/or C₅ olefin containing stream, more preferably a C₅ olefin containing stream.

The feed may also comprise one or more paraffins. As used herein, "paraffins" refers to any of the saturated hydrocarbons having the formula CₙH₂ₙ₊₂ wherein C, H and n are defined here above. The paraffins that may be present in the olefin feed typically have from 1 to 25 carbon atoms, such as from 1 to 15 carbon atoms, and conveniently at least 3 and no more than 6 carbon atoms. Examples of suitable paraffins include methane, ethane, propane, butane, pentane, hexane, isomers thereof and mixtures thereof. If present in the feed, the paraffins may have the same or a different number of carbon atoms as the olefins.

If present, the paraffin usually acts as a diluent. If used, the olefin feed may comprise at least 10%, at least 25%, at least 30%, at least 35%, or at least 40% paraffin, based upon the total volume of the feed. Alternatively stated, if used, the diluent may be present in the olefin feed in the range from 10% to 40%, alternatively, from 10% to 35%, and alternatively, from 20% to 35% based upon the total volume of the feed. The diluent may also be fed to the reactor(s) separately from the olefin feed. When fed separately, the diluent may be fed in amounts equivalent to those mentioned above, where the diluent is co-fed with the feed.

In a class of embodiments, the olefin containing feed comprises olefins selected from propene, butenes, pentenes, hexenes, their isomers, and mixtures thereof. The process of this invention is especially useful for the oligomerization of feeds comprising propene, butenes, pentenes, their isomers, and mixtures thereof. As used herein, "isomers" refers to compounds having the same molecular formula but different structural formula.

Additionally, the feed may comprise an oligomer (higher olefin), for example, a dimer, such as one provided by recycling a part of an olefin oligomerization product stream. As used herein, "oligomer(s)" or "oligomer product" refers to an olefin (or a mixture of olefins) made from a few light olefins. For example, oligomers include dimers, trimers, tetramers, obtained from two, three or four light olefins of the same number of carbon atoms, mixed oligomers, obtained from 2 or more olefins having different numbers of carbon atoms and mixtures thereof. In a class of embodiments, "oligomer(s)" refers to an olefin (or a mixture of olefins) having 20 carbon atoms or less, alternatively, 15 carbon atoms or less, such as 10 carbon atoms or less, alternatively, 9 carbon atoms or less, and conveniently, 8 carbon atoms or less.

In a class of embodiments, the feed comprises 30 wt% or more olefins, such as 40 wt% or more olefins, alternatively, 50 wt% or more olefins, alternatively, 60 wt% or more olefins, alternatively, 70 wt% or more olefins, and alternatively, 80 wt% or more olefins, based upon the total weight of the feed.

In any of the olefin oligomerization embodiments described herein, the feed should be totally free, or at least substantially free, of aromatic hydrocarbon compounds that consist solely of hydrogen and carbon atoms. In this context, "substantially free" means that the olefin feed contains 25 wt% or less, preferably 15 wt% or less, more preferably 10 wt% or less, such as 5 wt% or less, and most preferably 1 wt% or less aromatic hydrocarbon, based upon the total weight of the olefin feed.

Examples of suitable olefin feeds include untreated refinery streams such as Fluidized Catalytic Cracking (FCC) streams, steam cracker streams, coker streams, pyrolysis gasoline streams or reformates.

Examples of suitable C₃ olefin containing feedstreams include untreated C₃ rich refinery streams such as "dilute" or "refinery grade" propylene from a Fluidized Catalytic Cracker (FCC), C₃ rich stream from a steam cracker, from the production of "chemical grade" or "polymer grade" propylene, from refinery gas recovery units, from Propane Dehydrogenation Units, from Gas to Olefin (GTO) Units, or from Fisher-Tropsch Units, and C₃ rich return streams from polypropylene producing units. These C₃ streams may contain for example from 50 to 60 wt% of propylene, or 65 wt% or more, or 70 wt% or above such as 72 wt % or 75 wt % or even up to 79 wt %. The C₃ streams containing from 5 to 95 wt% of propylene are suitable
Examples of suitable C₄ olefin containing feeds include refinery feeds often referred to as Raffinate-1 (RAF-1), Raffinate-2 (RAF-2) or Raffinate-3 (RAF-3). Typically, Raffinate-1, Raffinate-2 and Raffinate-3 may be regarded as streams obtainable at various stages in the processing of crude C₄ streams obtained from petroleum refining processes. These streams are well known by the person skilled in the art.

In another embodiment, the feed comprises an FCC light olefin stream that typically comprises ethane, ethylene, propane, propylene, isobutane, n-butane, butenes, pentenes, pentanes, and other optional components, preferably in the amounts as disclosed hereinabove.

Examples of suitable C5 olefin feeds include FCC Light Naphtha streams, steam cracker C5 rich streams that have been treated for diene removal, C5 olefin containing streams from Gas to Olefin (GTO) Units, or Fisher-Tropsch Units. In this context, "Light Naphtha" is understood to mean a stream having a specific gravity in the range 0.65 to 0.73. An ASTM-D86 boiling point range between 35 and 125 °C and that contains a range of olefin, paraffin, diolefins and cyclic hydrocarbon compounds with carbons numbers typically in the range C5 to C8. More specifically, according to an embodiment, a so-called Light Light Catalytic Naphtha stream may be used. Such stream is characterized by a boiling point range of, for example, from 25 to 70 °C at atmospheric pressure and a specific gravity between 0.63 and 0.68 and contains at least 60 wt% C5 hydrocarbons.

These streams may comprise C₅ olefin components in the amounts as disclosed hereinabove. For example, an FCC Light Naphtha stream may comprise 50 wt% or more C₅ olefins, alternatively, 60 wt% or more C₅ olefins, alternatively, 70 wt% or more C₅ olefins, and preferably, 80 wt% or more C₅ olefins, based upon the total weight of the olefin feed. Additional distillations may be required to achieve the desired C₅ olefin content. These ranges also apply to the other specific C₅ olefin containing streams disclosed above.

According to the present invention, any of the above-described feeds contains nitrogen containing compounds and other impurities acting as catalyst contaminants which must be removed to an acceptable level before the hydrocarbon feed undergoes a catalyzed reaction. In particular, the nitrogen containing compounds comprise nitriles. As used herein, "nitrile" is any organic compound that has a nitrile group (or -C≡N functional group). In the nitrile group, the carbon atom and the nitrogen atom are triple bonded together. As used herein, "acetonitrile" (ACN) is the chemical compound with formula CH₃CN. This colorless liquid is the simplest organic nitrile. As used herein, "propanenitrile", "propionitrile", or "ethyl cyanide" is a nitrile with the molecular formula C₂H₅CN and the terms may be used interchangeably. It is also a clear liquid. Preferably the nitrile removed is a C₂ to C₅ nitrile. In the most preferred embodiment the nitrile to be removed is any of acetonitrile and propionitrile. These compounds are especially toxic to oligomerization catalysts and their removal leads to significant catalyst life improvement.

According to the present invention, any of the above-described feeds optionally contains at least one of diolefins and/or cyclic olefins. These compounds may also act as catalyst contaminants and are removed by the adsorption process used in this invention. In particular the dienes are hydrocarbons containing two carbon double bonds. The dienes concerned by this invention include but are not limited to C₄ to C₁₀ dienes. The cyclic olefins concerned by this invention include but are not limited to C₄ to C₁₀ hydrocarbon ring having at least one carbon carbon double bond. The double bond is preferably part of the cycle. Typical cyclic compounds are cyclopentene, methylcyclohexene, cyclohexene and cycloheptene.

### Distillation and hydrogenation

Prior to contacting the hydrocarbon feed with the adsorbent, the feed may be subjected to distillation and/or hydrogenation in order to change and/or improve desired properties of the feed.

The distillation / hydrogenation process may be used to reduce the level of compounds having an affinity to adsorbents for nitrogen-containing compounds other than the nitrogen containing compounds themselves. In particular, such compounds may be oxygenates, sulphur containing compounds, water, dienes and mixtures thereof. In this context, as used herein, the term "oxygenates" includes oxygen containing organic compounds including but not necessarily limited to aliphatic alcohols, ethers, carbonyl compounds (aldehydes, ketones, carboxylic acids, carbonates, and the like). It is to be noted that water is not considered herein as an oxygenate. Thus, when reference is made therein to the level of oxygenates present in a feed, this level does not include water. The sulphur compounds may include, but are not limited to methanethiol, ethanethiol, di-methyl-sulfide, carbon-di-sulfide, propanethiol and thiophene. The dienes concerned by this invention include but are not limited to C₄ to C₁₀ dienes.

Prior to the at least one distillation step, the feed may comprise the following first levels of nitrogen containing and other compounds having an affinity to adsorbents for nitrogen containing compounds.

The first level of nitrogen containing compounds may be in the range of from 0.1 wt ppm to 100 wt ppm, such as 3 wt ppm or more, such as about 5 wt ppm or more, typically, 10 wt ppm or more, such as 20 wt ppm or more, and yet alternatively, 30 wt ppm or more up to 100 wt ppm, based on the amount of nitrogen present in the compounds relative to the total weight of the stream.

The first level of oxygenates may be in the range of from 0.1 wt ppm to 500 wt ppm, relative to the total weight of the stream.

The first level of sulphur containing compounds may be in the range of from 0.1 to 100 wt ppm, expressed as the amount of sulphur present in the compounds relative to the total weight of the stream.

The first level of water may be in the range of from 0.1 wt ppm to 300 wt ppm relative to the total weight of the stream.

The first level of dienes or cyclic olefins may be in the range of from 0.01 wt% to 5 wt% relative to the total weight of the stream.

The distillation is usually carried out through use of trayed columns, packed columns including structured packing, random packing or a combination of both, combinations of trays and packing, e.g., in divided wall columns.

The distilled fractions and or combination thereof are then passed over the adsorbent to reduce the level of nitrogen containing compounds present in the feed.

The specific selection and blending of fractions will usually depend on the details of the composition of the feed stream. Also, the details of subsequent processing for converting the hydrocarbon feed into a desired hydrocarbon product may influence the selection and blending of fractions obtained from distillation step. For example, oligomerization, alkylation, isomerization conversion processes may use different catalysts at different process conditions and with different catalyst performance targets which will have an impact on acceptable levels of impurities which in turn can be controlled by selecting and blending distillation fractions as described above.

The distillation is preferably carried out to eliminate the bottom and top fractions of the hydrocarbon feed so that the combined stream the fraction(s) obtained in the distillation represent the "heart cut" of the hydrocarbon feed. Particularly advantageous results may be obtained when the high boiling fraction are completely eliminated before the absorption step. The combined or blended fractions preferably have a boiling range of from 27°Cto 37°C Typically the initial boiling point is 27.5, more preferably of 28.2°C. The T95 of the combined or blended fraction is of at most 35°C typically at most 33°C.

### Hydrocarbon conversion

According to the second aspect, the present invention relates to process for converting a hydrocarbon feed contaminated with at least one nitrogen-containing compound into a hydrocarbon product, said process comprising the steps of:
i) providing a hydrocarbon feed stream contaminated with at least one nitrogen-containing compound;
ii) contacting the feed stream with an adsorbent to remove the nitrogen-containing compound(s) from the feed;
iii) contacting the feed stream having a reduced level of nitrogen-containing compounds with a catalyst in order to convert the feed stream into a hydrocarbon product;
iv) regenerating the adsorbent comprising contacting the adsorbent with a gas atmosphere; and,
v) optionally, repeating steps (i) to (iii) or steps (i) to (iv),
wherein the regeneration step is as defined above.

The process for converting the hydrocarbon feed into a hydrocarbon product concerned by this invention may be an isomerization, an alkylation, a hydrogenation, an aromatization or an oligomerization process, preferably such process is an isomerization or an oligomerization process.

Typically the process of the present invention is an olefin oligomerization process and the hydrocarbon feed is an olefin containing feed as described above. As used herein, "oligomerization process" refers to any process by which light olefins are linked together to form the oligomer(s) as defined herein. As used herein, the term "oligomerization conditions" refers to any and all those variations of equipment, conditions (e.g. temperatures, pressures, weight hourly space velocities etc.), materials, and reactor schemes that are suitable to conduct the oligomerization process to produce the oligomer(s) as known and applied in the art and discussed in more detail below.

In a preferred embodiment, the hydrocarbon feed comprises an olefin, wherein the olefin is preferably selected from the group consisting of C₃, C₄, C₅ and C₆ olefins and mixtures thereof, in particular C₃, C₄ and C₅ olefins.

In a preferred embodiment, the hydrocarbon product comprises an oligomerization product and the catalyst is an oligomerization catalyst comprising a material selected from the group consisting of zeolites, phosphoric acids, supported metal oxides and combinations thereof. Preferably, the oligomerization catalyst comprises a zeolite, in particular a zeolite selected from the group consisting of ZSM-22, ZSM-57, ZSM-5, ITQ-39 and combinations thereof.

In a preferred embodiment, the oligomerization product is further subjected to any of the following steps: fractionation, hydrogenation, hydroformylation, oxidation, carbonylation, etherification, epoxidation, hydration or a combination thereof.

As noted above, one or more catalysts may be used in oligomerization processes of several embodiments of the invention. Any catalyst may be used so long as it is suitable to oligomerize olefins. Both homogeneous and heterogeneous catalysts may be used.

An example of a homogeneous catalyst includes the IFP (now Axens) DIMERSOL processes which employ a Ni-based homogeneous catalyst. (See, for example, Y. Chauvin et al., Chemistry and Industry, 1974, pages 373-378 and US 3,655,810.) Additionally, US 4,225,743 discloses a homogeneous catalyst system consisting of a nickel (II) salt of octanoic acid, ethylaluminium dichloride, and a free fatty acid.

In contrast, several of the industrial processes use heterogeneous catalysts. Most of these catalysts belong to one of the following groups: a) mineral acids (e.g., sulfuric acid or phosphoric acid) on a support material (e.g., alumina or silica), b) zeolites or other aluminum silicates, "undoped" or "doped" by further metals, in particular, for example, with transition metals, and c) acidic ion exchange resins. Examples are described in US-A- 2004/0097773.

Heterogeneous catalysts may be divided into crystalline and amorphous (non-crystalline) catalyst categories. Crystalline catalysts include, without limitation, molecular sieve catalysts such as, for example, zeolite catalysts. Non-crystalline catalysts include, without limitation, solid acid catalysts such as, for example, solid phosphoric acid catalyst (sPa) and supported metal catalysts or supported metal oxide catalysts. Examples include without limitation phosphoric acid-kieselguhr, copper-pyrophosphate-charcoal, and phosphoric acid-coated quartz chips. Commercial processes include the CATPOLY™ Process (UOP and Sud Chemie) employing phosphoric acid on a silica support. Further catalysts are disclosed in the claims, description and examples of EP-A-0 570 411, U.S. Patent 6,025,533 and EP-A-1 694 617.

The OCTOL™ Process (UOP/Huels (now Evonik)) employing a nickel containing catalyst on a silica/alumina support is also useful. Amorphous silica alumina supports are useful and commonly utilized. Solid acid catalysts may be optionally practiced with promoters such as, for example, TaF5.

The catalysts utilized in the oligomerization processes may be any suitable zeolite catalyst(s) capable of oligomerizing olefins. Exemplary methods and materials are provided in WO 2012/033562, US 4,973,790, and US-A-2012/0022224.

The at least one zeolite catalyst may include a medium pore size molecular sieve having a Constraint Index of about 1 to about 12. Constraint Index and a method of its determination are described in, for example, US 4,016,218.

Preferably, the at least one zeolite catalyst is selected from at least one of ZSM-5, ZSM-11, ZSM-12, ZSM-18, ZSM-22, ZSM-23, ZSM-35, ZSM-38, ZSM-48, ZSM-50, ZSM-57, ITQ-39 and mixtures thereof. The at least one zeolite catalyst comprises molecular sieves having pores formed by 10-membered rings of tetrahedrally coordinated atoms, such as molecular sieves having the TON or MFS structure type.

Mixtures of two or more of catalysts may be used in the processes of the present invention. For example, the mixture may include ZSM-22 and ZSM-57 or ZSM-22 and ZSM-5 or ZSM-57 ZSM-5 and ITQ-39. The at least one zeolite catalyst may also be combined with other catalysts such as a solid phosphoric acid (sPa) catalyst or other acid catalysts.

The zeolite used in the oligomerization catalyst may have an average crystallite or particle size of up to 15 µm, such as within the range of from 0.01 to 6 µm, alternatively, from 0.05 to 5 µm, and alternatively, from 0.1 to 3 µm. As used herein, "average particle size" refers to the arithmetic average of the diameter distribution of the crystals on a volume basis.

Preferably, the zeolite is used in its proton, or acidic form. To obtain this form, an as-synthesized molecular sieve that has been obtained in an alkaline or alkaline-metal form is advantageously converted to its acid form, for example, by acid treatment, e.g., by HCl, acetic acid, etc. or by ion exchange, for example, ammonium ion exchange. Subsequently, it may undergo calcination before use. The calcined materials may be post-treated, such as by steaming.

The at least one zeolite catalyst may be produced by any suitable method known for the given type of zeolite.
It may be desirable to incorporate the molecular sieves or zeolites mentioned above with another material that is resistant to the temperatures and other conditions employed in the olefin oligomerization process. Thus the molecular sieves or zeolites may be used in the form of an extrudate with binder, where the molecular sieve or zeolite is dispersed within a conventional binder as disclosed hereabove in connection with the adsorbents.

In the context of oligomerization, suitable reaction conditions may include temperatures from about 80°C to about 350°C. Close to and above the upper end of the range, cracking rates increase and may predominate over the oligomerization reaction providing an upper limit to practical operation. More typically, the reaction temperature is from about 130°C to about 320°C, preferably from about 135°C to about 310°C, and even more preferably from about 160°C to about 270°C.

The pressure may be in the range of from about 400 psig to about 4000 psig (2860 to 27688 kPa), and alternatively, from about 500 psig to about 1500 psig (3550 to 10446 kPa). The olefin weight hourly space velocity based on catalyst, may be in the range of from about 0.1 hr⁻¹ to about 20 hr⁻¹ or from about 0.5 hr⁻¹ to about 5 hr⁻¹.

In one embodiment, process is conducted at a temperature of 80-350°C; an olefin weight hourly space velocity of 0.1-20 hr⁻¹, and a pressure of 2860-27688 kPa.

In another embodiment, the process is conducted at a temperature of 130-320°C; an olefin weight hourly space velocity of 0.5-5 hr⁻¹; and a pressure of 3550-10446 kPa.

Optionally, the olefin feed may also be hydrated (i.e., contacted with water) prior to oligomerization. In an embodiment, sufficient water is used to saturate the feed. In particular, the feed may comprise from about 0.01 to about 0.25, alternatively, from about 0.02 to about 0.20, and alternatively, from about 0.03 to about 0.10, mol% water based on the total hydrocarbon content of the feed. If desired and by way of example, the water content of the feed may be increased by passage through a thermostatted water saturator. The olefin feed used in the oligomerization step can therefore be wet or dry.

### Hydrocarbon products

The invention is particularly but not exclusively concerned with processes suitable for the production of C5 to C20 olefins boiling in the range of 30° to 310°C, preferably 30° to 300°C, more preferably 30° to 250°C, from propylene and/or butene and/or pentene containing feedstocks or their mixtures, though ethylene may be present as well. The oligomer product may be fractionated in a series of discrete products. In particular the invention is concerned with the production of the olefins shown in the following table.

| | Distillation Range (°C) | |
|---|---|---|
| Oligomer Products | ASTM D1078 | |
| | Initial Boiling Point | Dry Point |
| Pentenes | 30 | |
| Hexenes | 35 | 72 |
| Heptenes | 88 | 97 |
| Octenes | 114 | 126 |
| Nonenes | 135 | 143 |
| Decenes | 155 | 160 |
| Undecenes | 167 | 178 |
| Propylene Tetramers or Dodecenes | 175 | 225 |
| Tridecenes | 204 | 213 |

The oligomer products are useful in many applications and are the starting material for further processes. For example, the oligomer product may be polymerized to produce polyolefins that have application in the plastic industry and synthetic basestocks for lubricants. The oligomer product may be used in alkylation reactions for the product of surfactants. The oligomer products may be reacted with sulphur containing compounds to produce mercaptans. The oligomer product may undergo hydroformylation and subsequently hydrogenation to produce alcohols. The alcohols may be used in industry such as, for example, solvents, or be incorporated into the production of detergents/surfactants. The alcohols may further be used in many other areas of industry such as, for example, undergoing esterification to produce esters that have application as plasticizers. Oligomer products may be hydrogenated to produce a predominately paraffin product such as ISOPAR™.

Products could be streams suitable for blending into fuels dispositions including Mogas, distillate, diesel, jet fuel etc. from processes like EMOGAS (ExxonMobil Olefins to Gasoline), MODG (Mobil Olefins to Diesel and Gasoline).

### Examples

The examples illustrate that when using a process according to the invention, it is possible to significantly or even fully restore the initial capacity for the adsorption of nitrogen-containing compounds such as PCN after each adsorption/regeneration cycle. This result represents a considerable improvement in dealing with the potentially harmful effects of nitrogen-containing impurities, dienes and cyclic olefins present in hydrocarbon feed materials of interest.

In the following examples, the process of the present invention is illustrated by means of a number of tests using NaY extrudates as the adsorbent. In examples 1 and 2, the NaY extrudate were crushed and sieved to 0.4 to 0.6 mm size. The adsorbent used in examples 3 to 8 is the commercially Zeolite Na-Y available from Zeolyst as ZEOLYST™ CBV 100 CY (1.6) characterized by white rods of 1/16 inch diameter and by a ratio of zeolite (faujasite) to binder of 80:20. Before passing the feedstock over the adsorbent, the adsorbent was pretreated at 230°C for 24 hours in dry nitrogen.

Then, an adsorption cycle using the pretreated adsorbent was carried out. The adsorption took place in a tubular reactor at 40°C and a weight-hourly-based-velocity (WHSV) of 3.2 h⁻¹.

Regeneration is carried out in the same unit at conditions disclosed in the examples.

### Example 1

In this example, a feed stream having the following composition was passed over the NaY adsorbent.

| Component | wt% |
|---|---|
| 1-pentene | 98.6 |
| 1,3-pentadiene | 1 |
| Isoprene | 0.4 |
| PCN | 80 ppm |
| H₂O | 100 ppm |

Regeneration was carried at 230°C using decene as a regeneration stream at a WHSV of 3.2 h⁻¹.

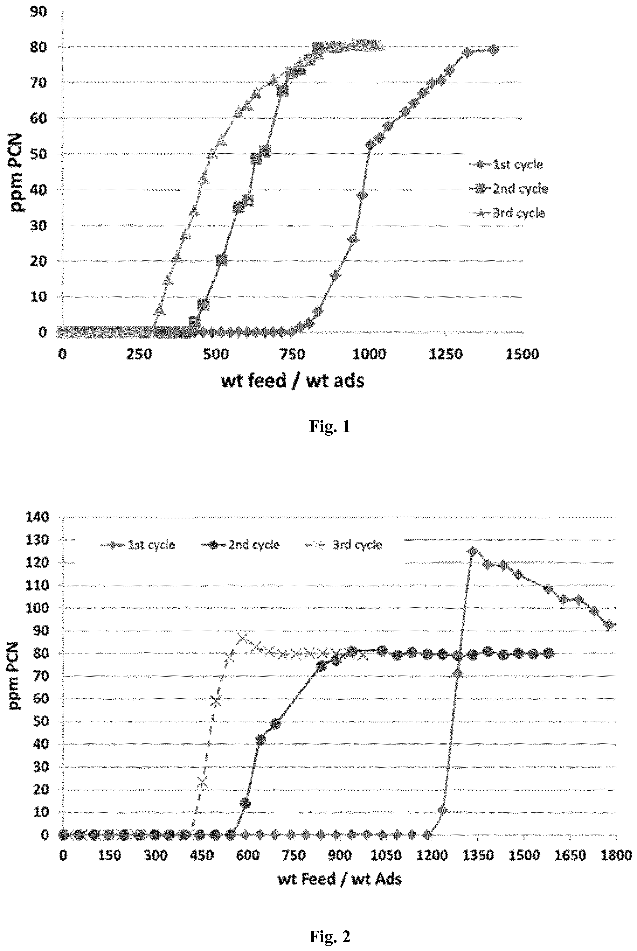

The results are shown in **Fig. 1** in terms of the increase in the level of PCN in the feed passing over the adsorbent, based on the amount of feed per amount of adsorbent (wt feed/wt ads). After passing a certain amount of feed over the adsorbent, as the adsorbent's capacity is progressively being lost due to adsorption of PCN and other species having an affinity to the adsorbent material such as dienes, the concentration of PCN in the feed stream raises toward the level of 80 ppm. In the first adsorption cycle, the breakthrough of PCN sets in at about 750 wt feed/wt ads. After regeneration in accordance with the procedure as defined above, but for a longer time (decene at 230°C for 24 h and at WHSV = 3.2 h⁻¹), when the feed is passed again over the adsorbent, the onset of the increase in the content of PCN is at about 450 wt feed/wt ads (2^{nd} cycle). After a further cycle of regeneration in accordance with the above conditions, the onset of breakthrough of PCN is even earlier, *i.e.,* at about 250 wt feed/wt ads (3^{rd} cycle). The earlier breakthrough of PCN illustrates that adsorption capacity is progressively lost when dienes are present in the olefin (1-pentene) feed and regeneration is carried out by using decene as the regeneration stream.

### Example 2

In this example, a feed stream having the following composition was passed over the NaY adsorbent.

| Component | wt% |
|---|---|
| 1-pentene | 99.4 |
| cyclopentene | 0.6 |
| PCN | 80 ppm |
| H₂O | 100 ppm |

Regeneration was carried out at 230°C using decene as a regeneration stream at a WHSV of 3.2 h⁻¹.

**Fig. 2** illustrates the results in terms of the increase in the level of PCN in the feed passing over the adsorbent, based on the amount of feed per amount of adsorbent (wt feed/wt ads). In the first cycle, the increase sets in at about 1200 wt feed/wt ads. After regeneration in accordance with the procedure as defined above, when the feed is passed again over the adsorbent, the onset of the increase in the content of PCN is at about 500 wt feed/wt ads (2^{nd} cycle). After a further cycle of regeneration in accordance with the above conditions, the onset of the increase in the level of PCN is even earlier, *i.e.,* at about 400 wt feed/wt ads. The earlier onset of the increase in the level in PCN illustrates that adsorption capacity is progressively lost when cyclopentene is present in the olefin (1-pentene) feed and regeneration is carried out by using decene as the regeneration stream.

### Example 3

This example presents a comparison between a regeneration procedure using decene at the conditions as detailed in Examples 1 and 2 and regeneration with a water-saturated nitrogen stream under the same temperature conditions. The feed stream used in this example had the following composition:

| Component | wt% |
|---|---|
| 1-pentene | 98.6 |
| 1,3-pentadiene | 1 |
| Isoprene | 0.4 |
| PCN | 80 ppm |
| H₂O | 100 ppm |

The water-saturated nitrogen stream was passed over the adsorbent at a flow of 25 ml/min, or about 12 h⁻¹ WHSV, a partial water pressure of 19.9 kPa (= vapor pressure of H₂O at 60°C) and at a temperature of 230°C.

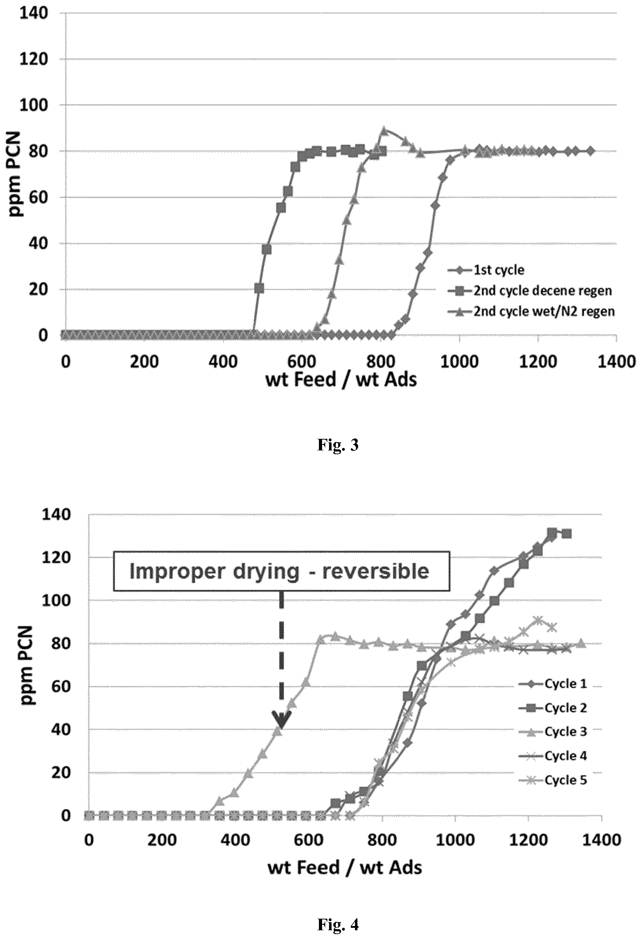

The results are shown in **Fig. 3** in terms of the increase in the level of PCN in the feed passing over the adsorbent, based on the amount of feed per amount of adsorbent (wt feed/wt ads). While the increase in the level of PCN when using fresh adsorbent sets in at about 800 wt feed/wt ads, after regeneration with decene the increase sets in at about 400 wt feed/wt ads. However, when regenerating in a water-saturated nitrogen stream, the onset of the increase in PCN occurs at about 600 wt feed/wt ads only. Thus, as compared to the use of a decene stream, under the same conditions, when using a water-saturated nitrogen stream, the loss in adsorption capacity after the 1^{st} cycle is reduced by about 50%.

### Example 4

This example illustrates a further embodiment of the use of a water-containing nitrogen regeneration stream in which a first regeneration step is carried out at 40 °C and a second regeneration step is carried out at 230 °C. The feed stream used in this example had the following composition:

| Component | wt% |
|---|---|
| 1-pentene | 98 |
| 1,3-pentadiene | 1 |
| Isoprene | 0.4 |
| Cyclopentene | 0.6 |
| PCN | 80 ppm |
| H₂O | 100 ppm |

Regeneration using a water-containing nitrogen stream at a flow F = 25 ml/min or 12 h⁻¹ WHSV was carried out in the same unit using a two-step procedure with a first regeneration step at a temperature of 40°C for 48h and a second regeneration step at 230°C for 24h followed by cooling down to 40°C in a stream of dry N₂. However, in the 3^{rd} cycle, the drying step was shortened. The vapor pressure (VP) of H₂O in the various regeneration cycles was:
1) VP (1^{st} cycle) = 2,6 kPa (vapor pressure of H₂O at 22°C)
2) VP (2^{nd} cycle) = 19,9 kPa (vapor pressure of H₂O at 60°C)
3) VP (3^{rd} cycle) = 19,9 kPa (vapor pressure of H₂O at 60°C)
4) VP (4^{th} cycle) = 19,9 kPa (vapor pressure of H₂O at 60°C)
5) VP (5^{th} cycle) = 2,6 kPa (vapor pressure of H₂O at 22°C)

The results are shown in **Fig. 4** in terms of the increase in the level of PCN in the feed passing over the adsorbent, based on the amount of feed per amount of adsorbent (wt feed/wt ads). The combination of a water-containing nitrogen stream and a two-step temperature treatment allows for full regeneration of the adsorbent after each cycle. The results obtained in cycle 3 are ascribed to an insufficient degree of drying after the high temperature regeneration step leading to presence of adsorbed water after cooling to 40 °C, which decreased NaY adsorption capacity for PCN. However, the initial capacity was fully recoverable in the following cycles.

Without wishing to be bound by a specific theory, it is believed that the use of the initial low temperature step allows dienes to desorb before they may oligomerize/polymerize as consequence of the high temperature step. The presence of water in the regeneration stream further supports desorbing the dienes. As water is the most polar molecule present in the system, it will compete for adsorption sites occupied by other molecules thereby displacing them away.

### Example 5 (not according to the invention)

This example illustrates an embodiment using a dry nitrogen regeneration stream in a first regeneration step at 40°C and a second regeneration step at 230°C. The feed stream used in this example had the following composition:

| Component | wt% |
|---|---|
| 1-pentene | 98.6 |
| Isoprene | 1.4 |
| PCN | 80 ppm |
| H₂O | 100 ppm |

Regeneration was conducted by using a dry nitrogen stream at a WHSV of 3.2 h⁻¹ at 40°C for 24h followed by regeneration at 230°C for 24h. The nitrogen flow F was 35 ml/min. After a total regeneration time of 48 h, the adsorbent was cooled down to 40°C while still passing dry nitrogen over the adsorbent.

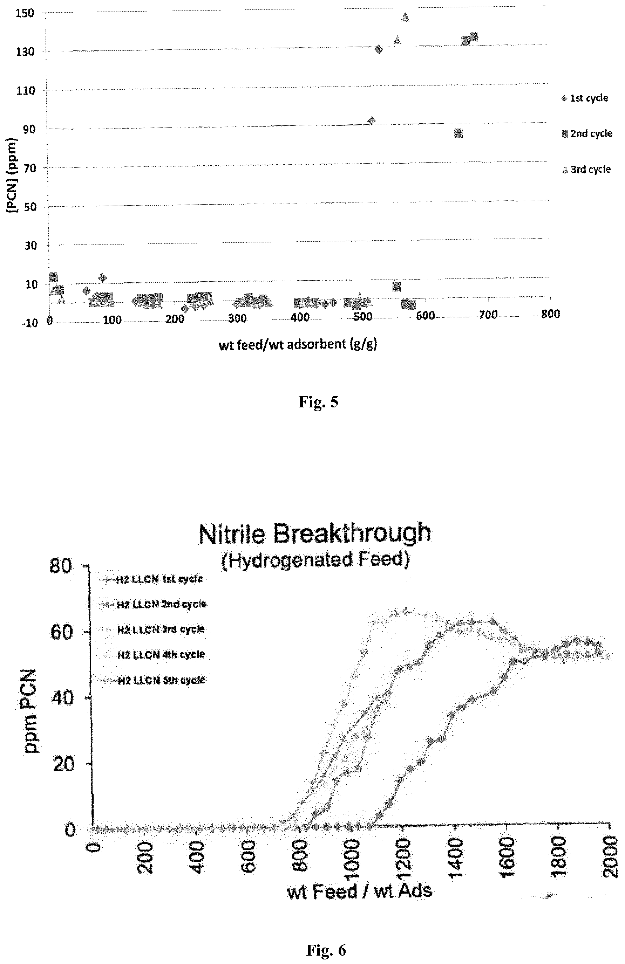

The results are shown in **Fig. 5** in terms of the increase in the level of PCN in the feed passing over the adsorbent, based on the amount of feed per amount of adsorbent (wt feed/wt ads). It can be seen that the original capacity of the adsorbent was restored when using the two step procedure as described in this example.

### Example 6

This example illustrates the process of the present invention when using a feed subjected to distillation and hydrogenation prior to contacting with a NaY adsorbent as specified above. The feed characteristics are summarized in Table the below.

Feed composition after distillation and hydrogenation.

| Composition | Hydrogenation Feed (Distilled LLCN) | Hydrogenation Product |
|---|---|---|
| < C4 sats | 0.0000 | 0.0054 |
| < C4 olefins | 0.0001 | 0.0071 |
| < C4 dienes | 0.0000 | 0.0000 |
| Isobutane | 0.0027 | 0.0062 |
| n-Butane | 0.0180 | 0.0272 |
| Iso butene | 0.0015 | 0.0020 |
| n-Butenes | 0.0448 | 0.0635 |
| C4 dienes | 0.0000 | 0.0000 |
| C4 cyclis (ol + sat) | 0.0000 | 0.0000 |
| Isopentane + 2,2 di-Me-Propane | 38.7109 | 36.1152 |
| n-pentane | 6.2382 | 7.9259 |
| Isopentenes | 27.0843 | 28.5341 |
| n-pentene | 26.1323 | 26.5060 |
| C5 dienes | 1.2445 | 0.2889 |
| C5 cyclis (ol + sat) | 0.4235 | 0.4746 |
| Hexenes | 0.0953 | 0.0417 |
| C6 sats | 0.0018 | 0.0056 |
| C6 cyclis (ol + sat) | 0.0000 | 0.0000 |
| C6 dienes | 0.0000 | 0.0000 |
| Heptenes | 0.0022 | 0.0002 |
| C7 sats | 0.0000 | 0.0000 |
| C7 cyclis (ol + sat) | 0.0000 | 0.0000 |
| C7 dienes | 0.0000 | 0.0000 |
| > C8 | 0.0000 | 0.0000 |
| Sum Unknowns | 0.0000 | 0.0000 |
| Total Oxy's (area %) | 0.0000 | 0.0000 |
| SUM hydrocarbons | 100.0000 | 100.0035 |

Specifically, the feed was distilled light liquid catalytic naphta (LLCN) obtained by the process described in the co-pending European application EP15150687.0. The selective hydrogenation was performed using a Pd on alumina catalyst LD-265 available from Axens under the following conditions:

| | | |
|---|---|---|
| Pressure | Bar | 17-18 |
| C5 rate | g/h | 2000 |
| H2/dienes ratio | Mol/mol | 0.5-1.6 |
| Temperature | C | 65 |
| Operating Mode | | Down flow |

After carrying out an initial adsorption cycle using the feed hydrogenation product as specified above, the adsorbent was subjected to regeneration. The 1^{st} to 4^{th} regeneration cycles were performed by using water-saturated nitrogen gas (partial pressure H₂O = 19.9 kPa (= vapor pressure of H₂O at 60°C)) at a flow of 25 ml/min or 12 h⁻¹ WHSV. In each cycle, the water-containing nitrogen gas stream was passed over the adsorbent at 40°C for 24 h followed by an increase to a temperature to 300°C for another period of 24 h followed by dry nitrogen at 300°C for 22 h. After a total period of 60 h, the adsorbent was cooled down to ambient temperature while still passing dry nitrogen over the adsorbent. The results are shown in Fig. 6 in terms of the increase in the level of PCN in the feed passing over the adsorbent, based on the amount of feed per amount of adsorbent (wt feed/wt ads). It can be seen that the initial capacity of the adsorbent can be restored to acceptable levels. Specifically, the onset of the increase in the level of PCN occurs only after significant amounts of feed have been passed over the adsorbent.

### Example 7

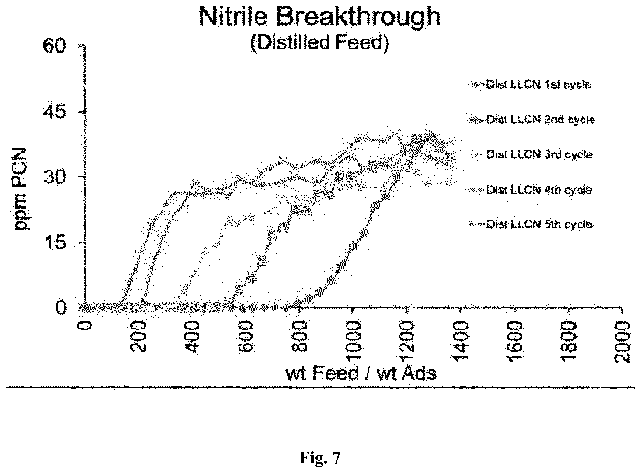

This example illustrates the process of the present invention when using a feed subjected to distillation prior to contacting with a NaY adsorbent as specified above. The feed characteristics were as stated hereinabove. The adsorbent was made and pretreated as described in Example 6. The regeneration procedure was as described in detail in Example 6. The results are shown in **Fig. 7** in terms of the increase in the level of PCN in the feed passing over the adsorbent, based on the amount of feed per amount of adsorbent (wt feed/wt ads). In comparison to the results reported in Example 6, it can be seen that the initial capacity of the adsorbent may not be restored to acceptable levels if too high a level of dienes is present in the feed. Then, as illustrated by Example 6, suitable hydrogenation ensures that the desired levels of regeneration are achieved.

### Example 8

This example illustrates treating (purifying) the spent nitrogen stream resulting from the regeneration process according to the present invention with low vapor pressure hydrocarbons to remove nitrogen-containing organic impurities such as PCN present in the stream.

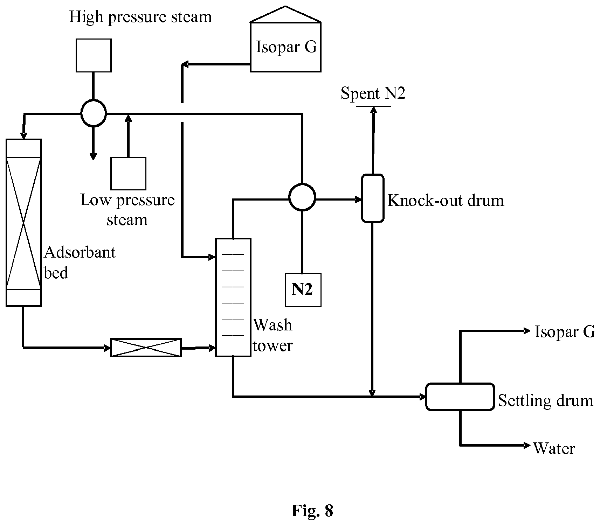

**Fig. 8** illustrates a regeneration process in accordance with the invention using water containing nitrogen. Water is added to nitrogen by injecting low pressure steam and the combined stream is used to regenerate the adsorbent. The spent nitrogen gas is washed with a saturated hydrocarbon with an initial boiling point of 162°C and final boiling point of 177°C commercialized under the denomination Isopar™ G by ExxonMobil to recover part of the water, the nitrogen containing compound and other compounds desorbed from the adsorbent. Water is removed from the saturated hydrocarbon in a settling drum and routed to an appropriate water treatment system (e.g. sour water stripper).

The washed nitrogen containing the saturated hydrocarbon is disposed of safely and within environmental permitting limits. To recover the saturated hydrocarbons from this nitrogen stream, the nitrogen stream is cooled and then disengaged from the saturated hydrocarbons in a knock-out drum before venting to atmosphere.

### Examples 9 and 10 (not according to the invention)

These examples illustrates the process of the invention when using a regeneration stream consisting essentially of saturated hydrocarbons
The adsorbent was identical to the one used in examples 1 and 2 above and the feed stream has the following composition.

| Composition | wt% |
|---|---|
| < C4 sats | 0.02 |
| < C4 olefins | 0.04 |
| < C4 dienes | 0.00 |
| Iso butane | 0.00 |
| n-Butane | 0.02 |
| Iso butene | 0.00 |
| n-Butenes | 0.13 |
| C4 dienes | 0.00 |
| C4 cyclis (ol + sat) | 0.00 |
| Isopentane + 2,2 di-Me-Propane | 36.22 |
| n-pentane | 7.52 |
| Isopentenes | 30.48 |
| n-pentene | 24.88 |
| C5 dienes | 0.016 |
| C5 cyclis (ol + sat) | 0.62 |
| Hexenes | 0.04 |
| C6 sats | 0.01 |

The regeneration stream is a saturated hydrocarbon with an initial boiling point of 162°C and final boiling point of 177°C commercialized under the denomination Isopar ™ G by ExxonMobil.

Regeneration in example 9 was carried out with a first regeneration step at 40°C for 5 hours, heating to 230°C at constant rate for 7 hours followed by a second regeneration step at 230°C for 16 hours before cooling down.

Regeneration in example 10 was carried out with a first regeneration step at 40°C for 5 hours, heating to 100°C at constant rate during 3 hours, a second step at 100°C for 6 hours and heating to 230°C at constant speed for 3 hours and a third step at 230°C for 3 hours before cooling down.

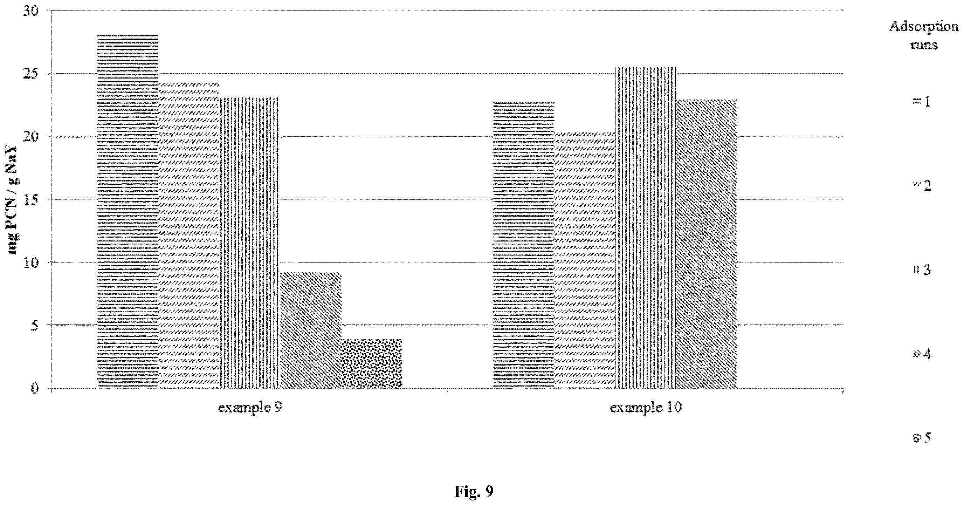

The results are shown in Fig.9 in terms of adsorption capacity of the adsorbent at 40°C over consecutive runs, each runs being separated by a regeneration treatment of the adsorbent. The results in example 10 show that the adsorption capacity is maintained over at least three successive regeneration runs when the regeneration includes an extra step at 100°C.

Without wishing to be bound by a specific theory it is believed that such step improves the desorption of diolefins and/or cyclic olefins while avoiding coking of the adsorbed diolefins and/or cyclic olefins on the adsorbent.

### Example 11 (not according to the invention)

This example illustrates the conversion of a hydrocarbon feed into a hydrocarbon product using a feed having a reduced level of nitrogen containing compounds obtained by using the adsorbent regenerated as disclosed in example 10 above. The hydrocarbon feeds with the composition as indicated in the table below are subjected to oligomerisation over a ZSM-57 catalyst.

### Composition of the feed

| Compound | wt% |
|---|---|
| Propane | 0.02 |
| Propene | 0.03 |
| iso-butane | 4.46 |
| n-butane | 8.85 |
| 1-butene | 42.41 |
| iso-butene | 2.33 |
| 1,3-butadiene | 0.17 |
| t-2-butene | 0 |
| c-2-butene | 0.02 |
| iso-pentane | 16.43 |
| 3-Me-butene-1 | 0.27 |
| n-pentane | 3.28 |
| pentene-1 | 1.46 |
| 1,4-pentadiene | 0.05 |
| 2-Me-butene-1 | 3.83 |
| pentene-2-t | 6.52 |
| pentene-2-c | 2.57 |
| Isoprene | 0.06 |
| 2-Me-butene-2 | 7.17 |
| Total C5 olefins | 21.82 |

Prior to contacting the catalyst, the feed is saturated with water by passing upward a hydrator at 30°C. The oligomerisation reactor is equipped with an axial thermowel containing a 3 point thermocouple. The reactor temperature was varied between 170°C and 260°C to maintain constant conversion while maintaining the pressure at 70 barg. Reactor space velocity (WHSV) was varied in the range of 3.0 to 5.0 h⁻¹. In example 11 and 12 butene conversion was controlled in the range of from 70 to 90%. In example 13 the propylene conversion was controlled at 90%.

The oligomerisation product is collected and analysed by gas chromatography equipped with a Pt on alumina catalyst to hydrogenate the olefins.

The C8 olefinic product has a branchiness of 2.1. The branchiness of the C9 olefinic product is of 2.2.

In summary, the process of the present invention allows for largely or fully restoring the initial adsorbent capacity. This is associated with the advantage of reducing the downtime of a unit using an adsorbent because the adsorbent cycle time is constant and no progressive capacity loss is observed. This also allows for more flexible plant operation, *i.e.,* reloading adsorbent units with fresh or regenerated adsorbent. The process of the present invention has broad applicability. For example, it can be used in any process in which in the feed dienes and cyclodienes are present and/or in which there is a need for removing nitrogen-containing impurities such as nitriles such as PCN or ACN. Also, the process for treatment of the spent inert gas can be used in connection with any process in which nitrogen-containing impurities are removed via adsorption to fixed-bed adsorbents.

## Claims

1. A process for regenerating an adsorbent for nitrogen-containing compounds present in a hydrocarbon feed comprising the steps of
a) contacting the adsorbent with a stream comprising an inert gas and water at a temperature in the range of from 10 to 60°C,
b) followed by contacting the adsorbent with a stream comprising an inert gas and water at an elevated temperature in the range of from 200 to 260°C;
c) followed by cooling the adsorbent in an inert gas,
wherein the inert gas is selected from nitrogen, saturated hydrocarbons and mixtures thereof and wherein the streams used in steps (a) and (b) are water saturated inert gas.

2. The process of claim 1, wherein the stream used in steps (a), and (b) comprises at least 96wt %of at least one inert gas, preferably nitrogen.

3. The process according to claim 1 or claim 2, wherein the process further comprises contacting the adsorbent from step (b) with an inert gas at a temperature in the range of from 200 to 250°C in order to remove water from the adsorbent before cooling according to step (c).

4. The process according to claim 1 or 2, wherein the stream used in steps (a) and (b) comprises at least 99.5 wt% nitrogen gas and the nitrogen gas resulting from the regeneration may be washed with a hydrocarbon having a low vapor pressure.

5. The process according to any of claim 1 or 2, wherein the stream used in steps (a) and (b) comprises at least 99 wt% of saturated hydrocarbons having from 1 to 16 carbon atoms.

6. The process of claim 4, wherein the step between steps (a) and (b) is carried out at a temperature in the range of 80 to 110°C.

7. The process according to any of the preceding claims, wherein the hydrocarbon feed contaminated with at least one nitrogen containing compound is an olefins containing feed also containing diolefins and/or cyclic olefins and is preferably subjected to selective hydrogenation to reduce the content of diolefins and/or cyclic olefins before contact with the adsorbent

8. The process according to any of the preceding claims, wherein the adsorbent comprises a zeolite having faujasite structure.

9. The process according to any of the preceding claims, wherein the elevated temperature used in step (b) is in the range of from 220 to 240°C.

10. The process according to any of the preceding claims, wherein water-containing nitrogen gas is provided in the form of a stream **characterized by** an inert gas flow of from 1 to 25 h⁻¹ WHSV and a water partial pressure of from 1 to 20 kPa.

11. The process according to any of the preceding claims, wherein the regeneration steps are conducted for a total period of from 12 to 144 hours, each of steps (a) to (c) being conducted for a period of from 12 to 48 hours.

12. The process according to any of the preceding claims, wherein no further steps are conducted that contribute to regeneration of the adsorbent.

13. The process according to any of the preceding claims, wherein the at least one nitrogen containing compound is a nitrile, such as propionitrile (PCN).

14. A process for converting a hydrocarbon feed contaminated with at least one nitrogen-containing compound into a hydrocarbon product, said process comprising the steps of:
i) providing a hydrocarbon feed stream contaminated with at least one nitrogen-containing compound;
ii) contacting the feed stream with an adsorbent to remove the nitrogen-containing compound(s) from the feed;
iii) contacting the feed stream having a reduced level of nitrogen-containing compounds with a catalyst in order to convert the feed stream into a hydrocarbon product;
iv) regenerating the adsorbent; and,
v) optionally, repeating steps (i) to (iii) or steps (i) to (iv),
wherein the regeneration step (iv) is as defined in any one of the preceding claims.

15. The process according to claim 13, wherein the hydrocarbon feed is an olefin containing feed further containing at least one of diolefins or cyclic olefins.

16. The process according to claim 14, wherein the olefin is selected from the group consisting of C₃, C₄, C₅ and C₆ olefins and mixtures thereof, in particular C₃, C₄ and C₅ olefins, the hydrocarbon product comprises an oligomerization product, and the oligomerization catalyst comprises a material selected from the group consisting of zeolites, phosphoric acids, supported metal oxides and combinations thereof.

## Patentansprüche

1. Verfahren zum Regenerieren eines Adsorbens für stickstoffhaltige Verbindungen, die in einem Kohlenwasserstoffeinsatzmaterial vorhanden sind, das die Schritte umfasst, in denen
a) das Adsorbens mit einem Strom, der Inertgas und Wasser umfasst, bei einer Temperatur im Bereich von 10 bis 60°C in Kontakt gebracht wird,
b) nachfolgend das Adsorbens mit einem Strom, der Inertgas und Wasser umfasst, bei einer erhöhten Temperatur im Bereich von 200 bis 260°C in Kontakt gebracht wird,
c) nachfolgend das Adsorbens in Inertgas gekühlt wird,
wobei das Inertgas ausgewählt ist aus Stickstoff, gesättigten Kohlenwasserstoffen und Mischungen davon, und wobei die in den Schritten (a) und (b) verwendeten Ströme wassergesättigtes Inertgas sind.

2. Verfahren nach Anspruch 1, bei dem der in den Schritten (a) und (b) verwendete Strom mindestens 96 Gew.% von mindestens einem Inertgas umfasst, vorzugsweise Stickstoff.

3. Verfahren nach Anspruch 1 oder Anspruch 2, das des Weiteren umfasst, dass das Adsorbens aus Schritt (b) bei einer Temperatur im Bereich von 200 bis 250°C mit Inertgas in Kontakt gebracht wird, um Wasser aus dem Adsorbens zu entfernen, bevor gemäß Schritt (c) gekühlt wird.

4. Verfahren nach Anspruch 1 oder 2, bei dem der in den Schritten (a) und (b) verwendete Strom mindestens 99,5 Gew.% Stickstoffgas umfasst und das aus der Regenerierung resultierende Stickstoffgas mit Kohlenwasserstoff mit niedrigem Dampfdruck gewaschen werden kann.

5. Verfahren nach einem der Ansprüche 1 oder 2, bei dem der in den Schritten (a) und (b) verwendete Strom mindestens 99 Gew.% gesättigte Kohlenwasserstoffe mit 1 bis 16 Kohlenstoffatomen umfasst.

6. Verfahren nach Anspruch 4, bei dem der Schritt zwischen den Schritten (a) und (b) bei einer Temperatur im Bereich von 80 bis 110°C durchgeführt wird.

7. Verfahren nach einem der vorhergehenden Ansprüche, bei dem das mit mindestens einer stickstoffhaltigen Verbindung verunreinigte Kohlenwasserstoffeinsatzmaterial Olefine enthaltendes Einsatzmaterial ist, das auch Diolefine und/oder cyclische Olefine enthält und vorzugsweise selektiver Hydrierung unterzogen wird, um vor dem Kontakt mit dem Adsorbens den Gehalt an Diolefinen und/oder cyclischen Olefinen zu reduzieren.

8. Verfahren nach einem der vorhergehenden Ansprüche, bei dem das Adsorbens Zeolith mit Faujasitstruktur umfasst.

9. Verfahren nach einem der vorhergehenden Ansprüche, bei dem die in Schritt (b) verwendete erhöhte Temperatur im Bereich von 220 bis 240°C liegt.

10. Verfahren nach einem der vorhergehenden Ansprüche, bei dem wasserhaltiges Stickstoffgas in Form eines Stroms bereitgestellt wird, der durch einen Inertgasfluss von 1 bis 25 h⁻¹ WHSV und einen Partialdruck des Wassers von 1 bis 20 kPa gekennzeichnet ist.

11. Verfahren nach einem der vorhergehenden Ansprüche, bei dem die Regenerationsschritte für einen Gesamtzeitraum von 12 bis 144 Stunden durchgeführt werden, wobei jeder der Schritte (a) bis (c) für einen Zeitraum von 12 bis 48 Stunden durchgeführt wird.

12. Verfahren nach einem der vorhergehenden Ansprüche, bei dem keine weiteren Schritte durchgeführt werden, die zur Regeneration des Adsorbens beitragen.

13. Verfahren nach einem der vorhergehenden Ansprüche, bei dem die mindestens eine stickstoffhaltige Verbindung ein Nitril ist, wie Propionitril (PCN).

14. Verfahren zum Umwandeln eines mit mindestens einer stickstoffhaltigen Verbindung verunreinigten Kohlenwasserstoffeinsatzmaterials in Kohlenwasserstoffprodukt, welches die Schritte umfasst, in denen:
i) ein Kohlenwasserstoffeinsatzmaterialstrom bereitgestellt wird, der mit mindestens einer stickstoffhaltigen Verbindung verunreinigt ist,
ii) der Einsatzmaterialstrom mit Adsorbens in Kontakt gebracht wird, um die stickstoffhaltige(n) Verbindung(en) aus dem Einsatzmaterial zu entfernen,
iii) das Einsatzmaterial mit einem reduzierten Niveau an stickstoffhaltigen Verbindungen mit Katalysator in Kontakt gebracht wird, um den Einsatzmaterialstrom in Kohlenwasserstoffprodukt umzuwandeln,
iv) das Adsorbens regeneriert wird, und
v) gegebenenfalls die Schritte (i) bis (iii) oder Schritte (i) bis (iv) wiederholt werden,
wobei der Regenerationsschritt (iv) wie in einem der vorhergehenden Ansprüche definiert ist.

15. Verfahren nach Anspruch 13, bei dem das Kohlenwasserstoffeinsatzmaterial olefinhaltiges Einsatzmaterial ist, das des Weiteren mindestens eines von Diolefinen oder cyclischen Olefinen enthält.

16. Verfahren nach Anspruch 14, bei dem das Olefin ausgewählt ist aus der Gruppe bestehend aus C₃-, C₄-, C₅- und C₆-Olefinen und Mischungen davon, insbesondere C₃-, C₄- und C₅-Olefinen, das Kohlenwasserstoffprodukt Oligomerisierungsprodukt umfasst, und der Oligomerisierungskatalysator Material ausgewählt aus der Gruppe bestehend aus Zeolithen, Phosphorsäuren, geträgerten Metalloxiden und Kombinationen davon umfasst.

## Revendications

1. Procédé pour régénérer un adsorbant pour composés contenant de l'azote présent dans une alimentation hydrocarbonée, comprenant les étapes suivantes :
a) la mise en contact de l'adsorbant avec un courant comprenant un gaz inerte et de l'eau à une température dans la plage allant de 10 à 60°C,
b) suivie par la mise en contact de l'adsorbant avec un courant comprenant un gaz inerte et de l'eau à une température élevée dans la plage allant de 200 à 260 °C ;
c) suivie par le refroidissement de l'adsorbant dans un gaz inerte,
le gaz inerte étant choisi parmi l'azote, les hydrocarbures saturés et leurs mélanges, et les courants utilisés dans les étapes (a) et (b) étant un gaz inerte saturé d'eau.

2. Procédé de la revendication 1, dans lequel le courant utilisé dans les étapes (a) et (b) comprend au moins 96% en poids d'au moins un gaz inerte, de préférence l'azote.

3. Procédé selon la revendication 1 ou la revendication 2, dans lequel le procédé comprend en outre la mise en contact de l'adsorbant de l'étape (b) avec un gaz inerte à une température dans la plage allant de 200 à 250°C afin d'éliminer de l'eau de l'adsorbant avant le refroidissement selon l'étape (c).

4. Procédé selon la revendication 1 ou 2, dans lequel le courant utilisé dans les étapes (a) et (b) comprend au moins 99,5 % en poids d'azote gazeux et l'azote gazeux résultant de la régénération peut être lavé avec un hydrocarbure ayant une faible pression de vapeur.

5. Procédé selon l'une quelconque des revendications 1 ou 2, dans lequel le courant utilisé dans les étapes (a) et (b) comprend au moins 99 % en poids d'hydrocarbures saturés contenant de 1 à 16 atomes de carbone.

6. Procédé de la revendication 4, dans lequel l'étape entre les étapes (a) et (b) est réalisée à une température dans la plage allant de 80 à 110 °C.

7. Procédé selon l'une quelconque des revendications précédentes, dans lequel l'alimentation hydrocarbonée contaminée avec au moins un composé contenant de l'azote est une alimentation contenant des oléfines contenant également des dioléfines et/ou des oléfines cycliques et est de préférence soumise à une hydrogénation sélective afin de réduire la teneur en dioléfines et/ou oléfines cycliques avant la mise en contact avec l'adsorbant.

8. Procédé selon l'une quelconque des revendications précédentes, dans lequel l'adsorbant comprend une zéolithe ayant une structure faujasite.

9. Procédé selon l'une quelconque des revendications précédentes, dans lequel la température élevée utilisée dans l'étape (b) est dans la plage allant de 220 à 240 °C.

10. Procédé selon l'une quelconque des revendications précédentes, dans lequel l'azote gazeux contenant de l'eau est fourni sous la forme d'un courant **caractérisé par** un débit de gaz inerte de 1 à 25 h⁻¹ WHSH et une pression partielle d'eau de 1 à 20 kPa.

11. Procédé selon l'une quelconque des revendications précédentes, dans lequel les étapes de régénération sont réalisées pendant une période totale allant de 12 à 144 heures, les étapes (a) à (c) étant chacune réalisées pendant une période allant de 12 à 48 heures.

12. Procédé selon l'une quelconque des revendications précédentes, dans lequel aucune étape supplémentaire qui contribue à la régénération de l'adsorbant n'est réalisée.

13. Procédé selon l'une quelconque des revendications précédentes, dans lequel ledit au moins un composé contenant de l'azote est un nitrile, tel que le propionitrile (PCN).

14. Procédé pour convertir une alimentation hydrocarbonée contaminée avec au moins un composé contenant de l'azote en un produit hydrocarboné, ledit procédé comprenant les étapes suivantes :
i) la fourniture d'un courant d'alimentation hydrocarbonée contaminé avec au moins un composé contenant de l'azote ;
ii) la mise en contact du courant d'alimentation avec un adsorbant pour éliminer le ou les composés contenant de l'azote de l'alimentation ;
iii) la mise en contact du courant d'alimentation ayant un niveau réduit de composés contenant de l'azote avec un catalyseur afin de convertir le courant d'alimentation en un produit hydrocarboné ;
iv) la régénération de l'adsorbant ; et
v) éventuellement, la répétition des étapes (i) à (iii) ou des étapes (i) à (iv),
l'étape de régénération (iv) étant telle que définie dans l'une quelconque des revendications précédentes.

15. Procédé selon la revendication 13, dans lequel l'alimentation hydrocarbonée est une alimentation contenant des oléfines contenant en outre des dioléfines et/ou des oléfines cycliques.

16. Procédé selon la revendication 14, dans lequel l'oléfine est choisie dans le groupe constitué par les oléfines en C₃, C₄, C₅ et C₆ et leurs mélanges, en particulier les oléfines en C₃, C₄ et C₅, le produit hydrocarboné comprend un produit d'oligomérisation, et le catalyseur de l'oligomérisation comprend un matériau choisi dans le groupe constitué par les zéolithes, les acides phosphoriques, les oxydes métalliques supportés et leurs combinaisons.
